# EUROPEAN PATENT APPLICATION

(11) **EP 3 967 752 A1**
(43) Date of publication of application: **16.03.2022**
(21) Application number: 20196306.3
(22) Date of filing: 15.09.2020
(51) Int. Cl.: C12N 5/0783, A61K 35/17, A61K 39/215, A61P 31/14

(54) **VIRUS SPECIFIC CYTOKINE-INDUCED KILLER CELLS**

(71) Applicant: Schmidt-Wolf, Ingo, 53127 Bonn (DE)
(72) Inventor: SCHMIDT-WOLF, Ingo, 53127 Bonn (DE)
(74) Representative: Letzelter, Felix Phillip

(57) **Abstract**

The present invention is directed to Virus specific Cytokine-Induced Killer cells (CIK), their use as a medicament and to a method for obtaining these Virus specific Cytokine-Induced Killer cells.

## Description

The present invention is directed to Virus specific Cytokine-Induced Killer cells (CIK), their use as a medicament and to a method for obtaining these Virus specific Cytokine-Induced Killer cells.

A viral disease usually occurs when an organism's body is invaded by pathogenic viruses, and infectious virus particles attach to and enter susceptible cells.

Mankind has been suffering from viral diseases since the beginning of time and since only a limited number of virus infections can currently be vaccinated against, the provision of medication against viral diseases is of major importance.

The newly discovered coronavirus known as severe acute respiratory syndrome coronavirus 2 (SARS-CoV-2), previously referred to by its provisional name 2019 novel coronavirus (2019-nCOV), is the seventh known coronavirus to infect humans.

Out of those seven coronaviruses SARS-CoV, MERS-CoV and SARS-CoV-2 have shown that they can cause severe disease. These viruses have caused large-scale pandemics in the last twenty years.

SARS-CoV-2 is an enveloped, non-segmented positive-sense single-stranded RNA virus and causes the respiratory illness Coronavirus disease 2019 (COVID-19).

Since its first documented emergence in late 2019 in China the virus has spread into an ongoing worldwide pandemic.

The scale of the COVID-19 pandemic shows the urgency of finding therapies for these kinds of viral infections. An effective therapy helps to reduce the number of mortalities. Furthermore, in view of such an effective therapy, less harsh epidemiological interventions like quarantines and lockdowns might be necessary, with all their negative economic and social effects on countries and societies.

The present invention addresses these needs.

It was one objective of the present invention to provide an antiviral medicament, in particular for the treatment of corona-viral diseases, especially for COVID-19.

This objective has been solved by Virus specific Cytokine-Induced Killer cells (CIK) according to claim 1, in particular by Virus specific Cytokine-Induced Killer cells (CIK) obtainable by co-culturing a CIK with at least one other population of eukaryotic cells that have been pulsed with viral antigens.

The ensuing description provides some embodiment(s) of the invention, and is not intended to limit the scope, applicability or configuration of the invention or inventions. Various changes may be made in the function and arrangement of elements without departing from the scope of the invention as set forth herein.

The terminology used in the description of the disclosure herein is for the purpose of describing particular embodiments only and is not intended to be limiting of the subject matter. As used in this description and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "includes," "including," "comprises," and/or "comprising," can also be understood as "consisting of" when used in this specification.

CIK cells have been first described by our group in Schmidt-Wolf I. G. H., Negrin R. S., Kiem H., Blume K. G., Weissman I. L. (1991) Use of SCID mouse/human lymphoma modelt o evaluate cytokine-induced killer cells with potent antitumor cell activity. J. Exp. Med., 174(1), 139-149, the disclosure of which is herein fully incorporated by reference.

For the generation of CIK cells, eukaryotic cells, such as Peripheral Blood Mononuclear Cells (PBMCs) are treated with a specific cytokine-cocktail for up to 21 days of in vitro culture. CIK cells are characterized as natural-killer (NK)-like T-cells, showing properties and markers of both NK and T cells. CIK cells are used in adaptive immunotherapy for the treating of cancer patients. CIK cell therapy has shown synergies when in used in combination with conventional cancer treatments like surgery or radiation therapy. The combination of CIK-cell therapy with chemotherapy has helped to overcome chemoresistance of cancer cells and reduce chemotherapy-associated adverse effects. Clinical studies have already shown promise for CIK-cell therapy in the treatment of various cancer-types including both acute and chronic leukemia, B cell lymphoma, gastric and lung cancers. Furthermore, CIK cells demonstrated only a very low cytotoxicity against non-malignant healthy cells. In a recent update, a total of 106 clinical trials including 10,225 patients were enrolled in the International Registry on CIK cells in cancer immunotherapy, of which 4,889 patients in over 30 distinct tumor entities were treated with CIK cells alone or in combination with conventional or novel therapies (Zhang Y et al.).

In Germany, CIK cells are licensed as a tumor vaccine by the Paul-Ehrlich-Institut (PEI).

By co-culturing CIK cells with at least one other population of eukaryotic cells, preferably dendritic cells (DC), that have been pulsed with viral antigens, the inventors surprisingly achieved to direct the specific cytotoxic reaction of CIK cells towards virus-infected cells instead of tumor cells.

Similar to CIK cell cancer therapy it is possible to exploit the specific cytotoxicity of CIK cells against virus-infected cells, without affecting surrounding healthy cells and tissues. By reducing the number of virus-infected cells with CIK cells the synthesis of new viruses can be limited leading to a decrease in total viral load.

Specificity of these cells is obtained by pulsing dendritic cells (DCs) with virus followed by coincubation of pulsed DCs with CIK cells.

In one embodiment of the invention, the at least one other population of eukaryotic cells comprises dendritic cells.

In another embodiment of the invention, the at least one other population of eukaryotic cells consist of dendritic cells.

DCs are pulsed with viral lysate prior to being put in co-culture with CIK cells, preferably from 1 to 10 days, preferably from 1 to 4 days, in particular for about 3 days.

In one embodiment of the invention, the CIK are derived from eukaryotic cells, preferably Peripheral Blood Mononuclear Cells, that have been treated with at least one of the cytokines IFNy, anti-CD3, IL1-β, IL-2, IL-4, GM-CSF and/or TNFa as described in "CIK cells have been first described by our group in Schmidt-Wolf I. G. H., Negrin R. S., Kiem H., Blume K. G., Weissman I. L. (1991) Use of SCID mouse/human lymphoma modelt o evaluate cytokine-induced killer cells with potent antitumor cell activity. J. Exp. Med., 174(1), 139-149)".

In another embodiment of the invention, the CIK are derived from eukaryotic cells, preferably Peripheral Blood Mononuclear Cells, that have been treated with the cytokines IFNy, anti-CD3, IL1-β, IL-2, IL-4, GM-CSF and TNFa.

In one embodiment of the invention, the CIK are derived from eukaryotic cells, preferably Peripheral Blood Mononuclear Cells, that have been treated with IFNy, IL1-β, anti-CD3 and IL-2, preferably in that order.

In one embodiment of the invention, the CIK are derived from eukaryotic cells, preferably Peripheral Blood Mononuclear Cells, that have been treated with IFNy on day 0, IL1-β on day 1, anti-CD3 on day 1 and IL-2 on day 1, preferably wherein, IL-2 is replenished every 3 days.

In one embodiment of the invention, the CIK are derived from eukaryotic cells, preferably Peripheral Blood Mononuclear Cells, that have been treated with IFNy in an amount of 100 to 500 U ml⁻¹, preferably about 300 U ml⁻¹ on day 0, IL1-β in an amount of 50 to 250 U ml⁻¹, preferably about 100 U ml⁻¹ on day 1, anti-CD3 in an amount of 10 to 100 ng ml⁻¹, preferably about 50 ng ml⁻¹ on day 1 and IL-2 in an amount of 100 to 500 U ml⁻¹, preferably about 300 U ml⁻¹ on day 1, preferably wherein, IL-2 is replenished every 3 days.

The treatment is preferably carried out for at least 3 days, preferably for up to 20 days, most preferably for about 14 days, such as 3-20 days, such as 3-14 days, such as 12-14 days.

In one embodiment, the CIK cell generation is complete after two weeks of maturation.

These treatments refer to the treatment of the CIK, before they are co-cultured with the at least one other population of eukaryotic cells that have been pulsed with viral antigens.

During the period of co-culturing, the treatment with at least one of the cytokines IFNy, anti-CD3, IL1-β, IL-2, IL-4, GM-CSF and/or TNFa, preferably, with IFNy, anti-CD3, IL1-β, IL-2, IL-4, GM-CSF and TNFa, more preferably with IFNy, IL1-β, anti-CD3 and IL-2, preferably in that order, is preferably continued.

In one embodiment of the invention, the CIK are obtainable by co-culturing a CIK with at least one other population of eukaryotic cells that have been pulsed with viral antigens, wherein the cells of the at least one other population of eukaryotic cells have been treated with at least one of the cytokines IFNy, anti-CD3, IL1-β, IL-2, IL-4, GM-CSF and/or TNFa, preferably GM-SCF and IL-4, preferably in that order.

The at least one other population of eukaryotic cells preferably comprise or consist of dendritic cells.

In one embodiment of the invention, the CIK are obtainable by co-culturing a CIK with at least one other population of eukaryotic cells that have been pulsed with viral antigens, wherein the cells of the at least one other population of eukaryotic cells have been treated with GM-SCF and IL-4.

In one embodiment of the invention, the CIK are obtainable by co-culturing a CIK with at least one other population of eukaryotic cells that have been pulsed with viral antigens, wherein the cells of the at least one other population of eukaryotic cells have been treated with GM-SCF and IL-4.

In one embodiment of the invention, the CIK are obtainable by co-culturing a CIK with at least one other population of eukaryotic cells that have been pulsed with viral antigens, wherein the cells of the at least one other population of eukaryotic cells have been treated with GM-SCF on day 0 and IL-4 on day 0.

In one embodiment of the invention, the CIK are obtainable by co-culturing a CIK with at least one other population of eukaryotic cells that have been pulsed with viral antigens, wherein the cells of the at least one other population of eukaryotic cells have been treated with GM-SCF in an amount of 500 to 1000 U ml⁻¹, preferably about 750 U ml⁻¹ on day 0 and with IL-4 in an amount of 250 to 750 U ml⁻¹, preferably about 500 U ml⁻¹ on day 0.

The treatment is preferably carried out for up to 3 days, preferably for 1 day, such as 1 hour to 3 days, such as 12 hours to 3 days, such as 12 hours to 1 day.

These treatments refer to the treatment of the at least one other population of eukaryotic cells, before they are co-cultured with CIK and before they were pulsed with viral antigens.

During the period of co-culturing, the treatment with at least one of the cytokines IFNy, anti-CD3, IL1-β, IL-2, IL-4, GM-CSF and/or TNFa, preferably, with IFNy, anti-CD3, IL1-β, IL-2, IL-4, GM-CSF and TNFa as described above is preferably continued.

In one embodiment of the invention the CIK and the at least one other population of eukaryotic cells that have been pulsed with viral antigens are cultured separately for up to 3 days, preferably for up to 20 days, most preferably for about 14 days before the co-culturing.

In another embodiment of the invention the at least one other population of eukaryotic cells is pulsed with viral antigens after 1 to 20 days, preferably after 4 days of individual culturing.

In one embodiment, the at least one other population of eukaryotic cells is pulsed with viral antigen on day 1 after treatment with GM-SCF and IL-4 as described above.

In one embodiment, the at least one other population of eukaryotic cells is pulsed with viral antigen on day 1 after treatment with GM-SCF and IL-4 as described above and is carried out for up to 7 days, preferably for up to 3 days, so that the at least one other population of eukaryotic cells is pulsed with viral antigen on day 1 after treatment with GM-SCF and IL-4 as described above and the pulsing with viral antigen is carried out until day 4.

Preferably, TNF-α is added on day 5, preferably in an amount of 250 to 750 U ml⁻¹, preferably about 500 U ml⁻¹.

The viral antigen preferably comprises viral lysate.

The viral antigen preferably comprises viral antigen from a coronavirus.

The viral antigen preferably comprises viral lysate from a coronavirus.

The viral antigen preferably comprises viral antigen from SARS-CoV-2.

The viral antigen preferably comprises viral lysate from SARS-CoV-2.

Corona viruses are widely distributed in humans and other mammals. In humans, the human corona viruses E229, OC43, NL63 and HKU1 are particularly important as pathogens of respiratory infections. The SARS coronavirus (2002) and the MERS coronavirus (2012) as well as the SARS-CoV-2 that induces severe diseases of the lower respiratory tract (COVID-19 = coronavirus disease 2019).

In one embodiment of the invention the at least one other population of eukaryotic cells that have been pulsed with viral antigens are co-cultured with the CIK for 5 to 20 days, preferably for 14 days.

Preferably, IL-2 is added, preferably in an amount of 300 to 900 U ml⁻¹, preferably about 600 U ml⁻¹ and preferably and replenished every 3rd day.

The invention is further directed to a virus specific CIK as described above for use as a medicament.

In another embodiment, the invention is directed to a virus specific CIK as described above for use in the treatment of viral diseases.

In another embodiment, the invention is directed to a virus specific CIK as described above for use in the treatment of corona-viral diseases.

In another embodiment, the invention is directed to a virus specific CIK as described above for use in the treatment of COVID-19.

All definitions and preferred embodiments that are disclosed in connection with the virus specific CIK apply mutatis mutandis to the use of the virus specific CIK as a medicament, in particular for use in the treatment of viral diseases, in particular corona-viral diseases, especially COVID-19.

The invention is further directed to a method for preparing Virus specific Cytokine-Induced Killer cells, comprising the step of co-culturing a CIK with at least one other population of eukaryotic cells that have been pulsed with viral antigens.

In another embodiment of the method of the invention the at least one other population of eukaryotic cells comprises Dendritic cells that have been pulsed with viral antigens from a coronavirus, in particular with viral antigens of SARS-CoV-2.

All definitions and preferred embodiments that are disclosed in connection with the virus specific CIK apply mutatis mutandis to the method for preparing Virus specific Cytokine-Induced Killer cells.

In one embodiment of the method of the invention PBMCs are isolated from buffy-coats derived from healthy donors. Both CIK cells and DCs are cultured separately for 7 days. During this period CIK cells and DCs are being treated with the specific cytokines IFNy, anti-CD3, IL1-β, IL-2, IL-4, GM-CSF and TNFa in order to induce their proliferation, differentiation and activation. Preferably CIK cells are derived from PBMCs by treatment with IFNy, IL1-β, anti-CD3 and IL-2 as described above.

Before pulsing with viral antigen, DC are preferably treated with GM-SCF and IL-4 as described above.

DCs are then pulsed with inactivated viral material, preferably with inactive SARS-CoV2-virus, preferably with SARS-CoV2-virus lysate at day 4 to provide them with virus-specific antigens.

From day 8 both CIK and DC are co-cultured until their maturation at day 14 of culture, to obtain virus specific CIK.

CIK cells with SARS-CoV2 specificity and conventional CIK cells can be phenotypically and functionally analyzed.

The success of culturing DCs and CIK cells can be determined by detecting CIK- and DC-specific surface markers via flow cytometry.

The reactive T cell subset which up-regulate CD137 can be further co-stimulated with anti-CD137, followed by expansion in G-Rex flasks under standard CIK culture condition as described by Luah et al. for CMV-specific CIK cells (Clin Immunol 2019 Aug;205:83-92. doi: 10.1016/j.clim.2019.06.007. Epub 2019 Jun 21).

The present invention is also directed to CIK cells for use in the treatment of corona-viral diseases in particular for use in the treatment of COVID-19.

The present invention is now further illustrated in the following examples. The examples should not be considered as limiting the invention in any way.

### Examples:

### Example 1:

### Mononuclear cell-isolation from whole blood

Blood from healthy donors was kindly provided by the Institute for Experimental Hematology and Transfusion Medicine of University Hospital of Bonn, Germany. Peripheral blood lymphocytes (PBL) were isolated from the whole blood by gradient density centifugation using Pancoll human (#P04-60500, PAN Biotech GmbH, Aidenbach, Germany). The whole blood was diluted 1:1 with 2% EDTA (#A3145,0500, Applichem GmbH, Darmstadt, Germany) in PBS (#P04-36500, PAN Biotech GmbH). 15 ml of the diluted blood was carefully layered on top of 30 ml of the Pancoll solution and centrifuged at 1000 x g for 30 mins without brakes at room temperature. The upper plasma layer was collected and sterile filtered with a 0.22 µm syringe-filter (#SLGP033RS, Sigma Aldrich, St. Louis, MO, USA). The white mononuclear cell layer underneath was collected, washed with PBS-EDTA and spinned down at 800 x g for 7 mins at room temperature. The pellet was incubated in red blood lysis buffer (#420301, Biolegend, San Diego, CA, USA) for 10 mins at 4°C. Afterwards the reaction was stopped by filling it up to 50 ml with PBS-EDTA and spinning it down at 800 x g for 7 mins. The pellet was then resuspended in RPMI medium (#P04-16500, PAN Biotech GmbH) supplemented with 20% of autologous filtered serum and 1% of PenStrep (#15140122, Thermo Fisher Scientific Inc, Waltham, MA, USA). The cell concentrations were determined manually and the dead cells were excluded using tryphan blue staining (#15250061, Thermo Fisher Scientific Inc.).

### PBL isolation from the mononuclear cells

45 x 10⁶ cells were seeded per well of a 6-well plate in 5 ml of RPMI medium supplemented with 20% of autologous serum and 1% of PenStrep. The cells were incubated for 1 h at 37°C with a 5% CO₂ atmosphere. Following the incubation, suspension cells were separated from adherent cells. The suspension cells were collected and spinned down at 800 x g for 7 min. The pellet was then resuspended in 20 ml of RPMI medium supplemented with 10% heat inactivated FCS (#F7524, Sigma Aldrich), 1% PenStrep and 250 mM of HEPES (#P05-01100, PAN Biotech GmbH). The FCS was previously heat-inactivated by 30 mins incubation at 56°C. The cells were seeded in a T-75 flask.

### CIK cell-generation from PBLs

The cells were stimulated with 2000 U/ml of IFN-γ (#11343536, ImmunoTools GmbH, Friesoythe, Germany) at day 0. The following day 100 U/ml of IL-1β (#11340013, ImmunoTools GmbH), 50 ng/ml of anti-CD3 antibody (#16-0037-81, Thermo Fisher Scientific Inc.) and 600 U/ml of IL-2 (#11340025, ImmunoTools GmbH) were added. The IL-2 was replenished every 3 days.

### DC culture and pulsing

After the suspension cells were removed from the 6-well plates, 2 ml of RPMI medium supplemented with 20% of autologous serum and 1% of PenStrep was added per well. The cells were stimulated with 750 U/ml of GM-CSF (#11343125, ImmunoTools GmbH) and 500 U/ml of IL-4 (#11340043, ImmunoTools GmbH) at day 0. The next day the cells were pulsed by adding SARS-CoV-2 lysate at a ratio of 1:500 (v/v) to the well. As a control 100 µg/ml of BSA (#05482, Sigma Aldrich) was added to another well. The pulsing was stopped at day 4 by exchanging the medium. At day 5 500 U/ml TNF-α (#11343015, ImmunoTools GmbH) was added.

### Pulsed DCs - CIK cells co-culture

At day 7 1.5 x 106 of CIK cells were added into the wells containing the DCs. The co-cultures were kept in 2 ml of RPMI medium, supplemented with 10% heat inactivated FCS, 1% PenStrep and 250 mM of HEPES. 600 U/ml of IL-2 was added and replenished every 3rd day of culture. 1 ml of fresh medium was added when required. At day 14 the mature CIK cells were harvested.

### CIK cells - SARS-CoV-2 infected Caco-2 cell lines co-culture

2 x 104 and 4 x 104 of Caco-2 cell line were seeded in 96-well plates in 100 µl of EMEM (#ATCC^{®} 30-2003^{™}, ATCC, Manassas, VA, USA). The next day the cells were infected with SARS-CoV-2 at a ratio of 1:5000 for 1 h. The remaining virus was removed and mature CIK cells were added to the infected Caco-2 cells with an E:T ratio of 5:1 for 4 x 104 Caco-2 cells and 10:1 for 2 x 104 Caco-2 cells respectively. The co-cultures were kept in 200 µl of OptiMEM (#31985070, Thermo Fisher Scientific Inc.) supplemented with 10% heat inactivated FCS and 1% PenStrep for 24 to 48 h at 37°C with 5% CO₂.

### Viral RNA isolation and cDNA synthesis

The 96-well plate was spinned down at 600 x g for 5 mins. The supernatant was collected for RNA isolation. The RNA isolation was perfomed using a Qiamp viral RNA mini kit (#52906, QIAGEN N.V., Venlo, Netherlands) according to the manufacturer's protocol. The elution of the bound RNA was done by adding 30 µl of nuclease-free water (#AM9938, Thermo Fishe Scientific Inc.) into the column.

The RNAs were used as templates for cDNA synthesis using SuperScript III SuperMix (#11752250, Thermo Fisher Scientific Inc.). The mixes were prepared per manufacturer's instruction with 8 µl of RNA template per reaction. The reactions were carried out in a Mastercycler^{®} Gradient Thermocycler (Eppendorf, Hamburg, Germany) starting at 25°C for 10 mins, 50°C for 1 h and 85°C for 5 mins. The cDNAs were stored at -20°C until further use.

### Amplification of viral cDNA

qPCR was performed to measure the viral RNA using a Mastercycler^{®}pro Thermocycler (Eppendorf). The assay was done using my-Budget 5x EvaGreen^{®} qPCR-Mix II (ROX) (#80-5801000, Bio-Budget GmbH, Krefeld, Germany) and predesigned SYBR Green SARS-CoV-2-specific primers (#KSPQ12012G, Sigma Aldrich). The forward and reverse primers were used at a concentration of 250 nM. The mixes were prepared according to the manufacturer's instruction, including 2 µl of both primers and 14 µl of cDNA template.

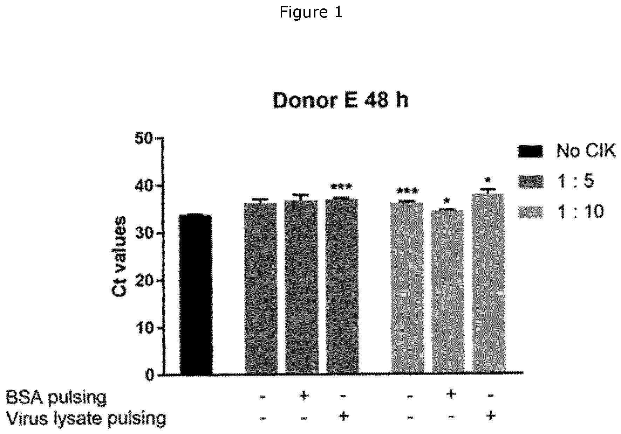

Figure 1 presents the data for this experiment. The first column (No CIK) represents the viral RNA-levels of CaCo2 cells that have been infected, but not treated with CIK cells. The following columns show viral RNA-levels for infected CaCo2 cells that have been co-cultured with CIK cells in ratios of 1:5 and 1:10 respectively. Furthermore, it compares the effects of CIK cells that have been cultured with DCs that have either not been specifically pulsed, pulsed with BSA and pulsed with viral lysate.

The data show that a a significant increase of the ct-values for the CIK-treated cultures can be detected, especially at the higher target:effector ratio of 1:10. A higher ct-value represents a lower concentration of viral RNA, indicating a lower viral load in total. The data show that CIK cells cause a decrease in the viral concentrations of infected cultures.

### Phenotyping of DCs and CIK cells

Flow cytometry analysis was perfomed for DCs and for CIK cells. Their surface marker expressions were measured using a BD FACS Canto II Cytometer (BD Biosciences, Franklin Lakes, NJ, USA). APC-CD80 (#344721, Biolegend,), FITC-CD11c (#301603, Biolegend), PerCP-Cy5-CD11c (#301623, Biolegend), PE-CD86 (#374205, Biolegend) and PE-Cy7-CD14 (#367111, Biolegend) conjugated antibodies were used to label the DCs.

The CIK cells were labeled with APC-CD3 (#317318, Biolegend), PE-CD56 (#362507, Biolegend) and PE-Cy7-CD8 antibodies (#344711, Biolegend). Dead cells were excluded by adding Hoechst 33258 (#99403, Sigma Aldrich) prior to the measurement. The data was analyzed with FlowJo v10 Software (BD Biosciences)

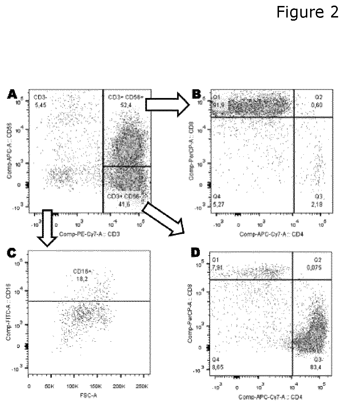

Figure 2: Phenotyping of effector cells on day 8 of CIK cell generation.

(A) Dot-plot of CD3 against CD56 of all viable cells. (B) Dot-plot of CD4 against CD8 of all CD3+CD56+ cells. (C) Dot-plot of CD16 of all CD3- cells. (D) Dot-plot of CD4 against CD8 of all CD3+CD56- cells.

Figure 2 shows exemplary data of the phenotyping of cells derived from a single donor prior to the addition of cytokines for CIK cell generation. Figure 2 (A) shows around 30 % CD3- cells and about 70 % CD3+ cells that are equally divided into CD56-positive and -negative cells. Figure 1 (B) shows that the majority of CD3+CD56+ cells are CD8+, while figure 1 (D) illustrates that most CD3+CD56- cells are CD4+. Figure 1 (C) presents around 70 % of all CD3- cells to be CD16+.

Figure 3: Phenotyping of effector cells on day 8 of CIK cell generation.

(A) Dot-plot of CD3 against CD56 of all viable cells. (B) Dot-plot of CD4 against CD8 of all CD3+CD56+ cells. (C) Dot-plot of CD16 of all CD3- cells. (D) Dot-plot of CD4 against CD8 of all CD3+CD56- cells.

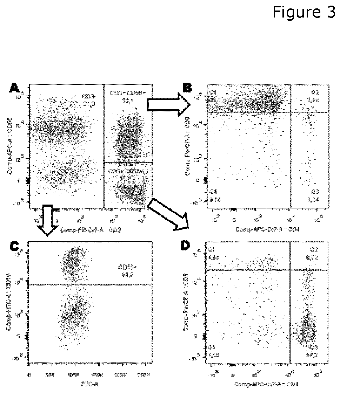

Figure 3 shows exemplary phenotyping of the same donor on day 8 of CIK cell generation. In figure 2 (A) it can be seen that over 90 % of all cells are CD3+, with an increase of CD3+CD56+ cells to about 50 % compared to roughly 40 % of CD3+CD56- cells. Similar to day 0, figures 2 (B) and (D) show that still most CD3+CD56+ cells are CD8+, while the majority of CD3+CD56- cells remains CD4+. Figure 2 (C) displays that the percentage of CD16+ cell out of all CD3-cells has decreased to around 18 %.

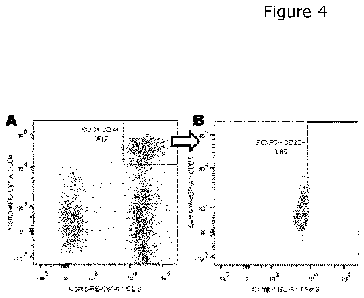

Figure 4: Phenotyping of regulatory T cells on day 0 of CIK cell generation.

(A) Dotplot of CD3 against CD4 of all viable cells. (B) Dot-plot of FOXP3 against CD25 of all CD3+CD4+ cells.

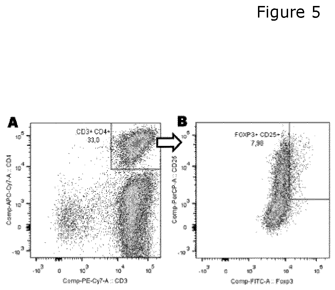

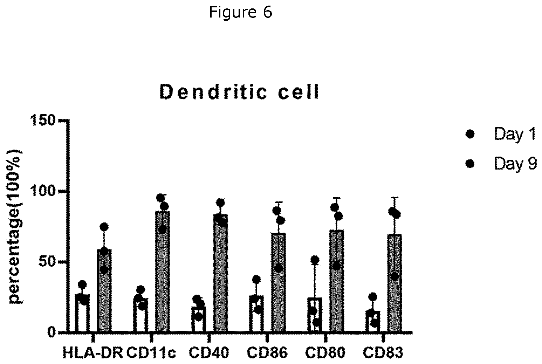

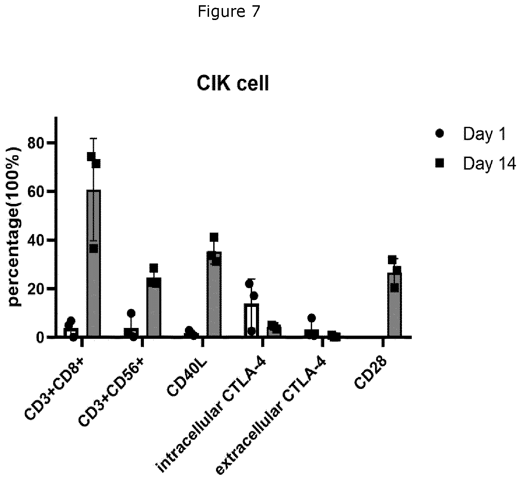

Figure 5: Phenotyping of regulatory T cells on day 0 of CIK cell generation.

(A) Dotplot of CD3 against CD4 of all viable cells. (B) Dot-plot of FOXP3 against CD25 of all CD3+CD4+ cells.

Figure 4 (A) shows that around 30 % of all cells are CD3+CD4+ at day 0 of CIK cell generation. In figure 4 (B) it is revealed that around 4 % of all CD3+CD4+ cells are positive for both FOXP3 and CD25.

Figure 5 presents the same markers as figure 3 just at day 8 of CIK cell generation. In Figure 5 (A) the percentage of CD3+CD4+ cells remains around 30 %, while the fraction of CD3- cells has apparently decreased.

Figure 5 (B) shows an increase especially in CD25, but also the fraction of FOXP3+CD25+ has increased to 8%.

Table 1 summarizes the data from both day 0 and day 8 of CIK cell generation for easier comparison. The top row of both day 0 and 8 represents the percentages of populations out of all viable cells, while the bottom row shows the percentage of sub-populations out of the corresponding top-row population in the same column. For example, the first column for day 0 shows 31.8 % of all viable cells to be negative for CD3 and 68.9 % of all CD3- cells to be positive for CD16.

**Table 1. Percentages of major populations measured by FACS analysis after 0 and 8 days of CIK generation. Exemplary data from one single donor.**

| | | | | | | |
|---|---|---|---|---|---|---|
| **Day 0** | **CD3-** | **CD3+CD56-** | | **CD3+CD56-** | | **CD3+CD4+** |
| | 31.8% | 35.1% | | 33.1% | | 30.7% |
| | **CD16+** | **CD4+** | **CD8+** | **CD4+** | **CD8+** | **FOXP3+CD25+** |
| | 68.9% | 87.2% | 4.7% | 3.2% | 85.3% | 3.7% |
| | | | | | | |
| **Day 8** | **CD3-** | **CD3+CD56-** | | **CD3+CD56-** | | **CD3+CD4+** |
| | 5.5% | 41.6% | | 52.4% | | 33.0% |
| | **CD16+** | **CD4+** | **CD8+** | **CD4+** | **CD8+** | **FOXP3+CD25+** |
| | 18.2% | 83.4% | 7.9% | 2.2% | 91.9% | 8.0% |

### Figure 6:

Phenotyping of dendritic cells on day 1 and day 9. The white bars show the expression of the respective makers on day 1, the grey bars show the expression of the respective makers on day 9.

### Figure 7:

Phenotyping of CIK cells on day 1 and day 14. The white bars show the expression of the respective makers on day 1, the grey bars show the expression of the respective makers on day 14.

### Example 2:

Further, analogously to example 1, the following protocols 1 and 2 have been tested.

### Protocol 1:

| | |
|---|---|
| Day 0: | - 750 U/ml GMCSF |
| | - 500 U/ml IL-4 |
| | - 45 x 106 cells / well (6 well plate) |
| Day 1; | - Pulsing (100 µg/ml) |
| Day 4: | - End pulsing / change medium |
| Day 5: | - 500 U/ml TNFa |
| Day 7: | - Co-culture with CIK cells (DC:CIK1:5) |
| | - In CIK-medium (600 U/ml IL-2) |
| | - Replenish IL-2 every 3 days |
| | - Phenotyping |
| Day 14: | - Maturation / Harvest |

### Protocol 2:

| | |
|---|---|
| Day 0: | - 1000 U/ml GMCSF |
| | - 1000 U/ml IL-4 |
| | - 25 x 106 cells / well (6 well plate) |
| Day 2: | - Change medium (incl. cytokines) |
| Day 5: | - Change medium (incl. cytokines) |
| Day 6: | - Pulsing (100 µg/ml) |
| Day 8: | - End pulsing / change medium |
| | - 1000 U/ml TNFa |
| Day 9: | - Co-culture with CIK cells (DC:CIK1:5) |
| | - In CIK-medium (600 U/ml IL-2) -Replenish IL-2 every 3 days |
| | - Phenotyping |
| Day 14: | - Maturation / Harvest |

Analogously to example 1, the cells have been phenotyped.

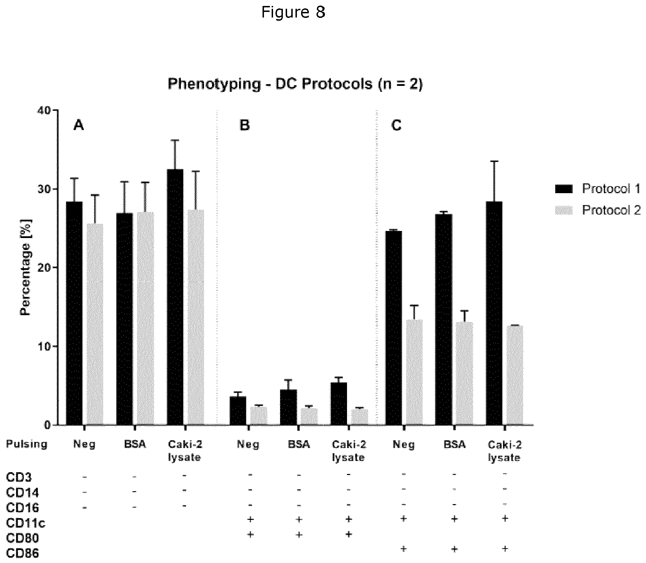

The results are summarized in figure 8.

Figure 8: DC-Protocol Phenotyping.

A: Percentage of CD3-CD14-CD16- cells out of all living cells. B: Percentage of CD11c+CD80+ cells out of all CD3-CD14-CD16- cells. C: Percentage of CD11c+CD86+ cells out of all CD3-CD14-CD16- cells.

## Claims

1. Virus specific Cytokine-Induced Killer cells (CIK), obtainable by co-culturing a CIK with at least one other population of eukaryotic cells that have been pulsed with viral antigens.

2. Virus specific CIK according to claim 1, wherein the at least one other population of eukaryotic cells comprises Dendritic cells.

3. Virus specific CIK according to claim 1 or 2, wherein the CIK are derived from eukaryotic cells, preferably Peripheral Blood Mononuclear Cells, that have been treated with at least one of the cytokines IFNy, anti-CD3, IL1-β, IL-2, IL-4, GM-CSF and/or TNFa, preferably with IFNy, IL1-β, anti-CD3 and IL-2, preferably for up to 7 days.

4. Virus specific CIK according to any of the preceding claims, wherein the cells of the at least one other population of eukaryotic cells have been treated with at least one of the cytokines IFNy, anti-CD3, IL1-β, IL-2, IL-4, GM-CSF and/or TNFa, preferably with GM-CSF and IL4, preferably for up to 7 days.

5. Virus specific CIK according to any of the preceding claims, wherein the CIK and the at least one other population of eukaryotic cells that have been pulsed with viral antigens are cultured separately for up to 7 days before the co-culturing.

6. Virus specific CIK according to any of the preceding claims, wherein the at least one other population of eukaryotic cells is pulsed with viral antigens after 4 days of individual culturing.

7. Virus specific CIK according to any of the preceding claims, wherein the at least one other population of eukaryotic cells that have been pulsed with viral antigens are co-cultured for up to 7 days, preferably after the at least one other population of eukaryotic cells that have been pulsed with viral antigens and the CIK are cultured separately for up to 7 days before the co-culturing.

8. Virus specific CIK according to any of the preceding claims, wherein the viral antigens comprise viral antigens of a coronavirus.

9. Virus specific CIK according to any of the preceding claims, wherein the viral antigens comprise viral antigens of SARS-CoV-2.

10. Virus specific CIK according to any of the preceding claims, for use as a medicament.

11. Virus specific CIK according to any of the preceding claims, for use in the treatment of viral diseases.

12. Virus specific CIK according to any of the preceding claims, for use in the treatment of corona-viral diseases.

13. Virus specific CIK according to any of the preceding claims, for use in the treatment of COVID-19.

14. Method for preparing Virus specific Cytokine-Induced Killer cells, comprising the step of co-culturing a CIK with at least one other population of eukaryotic cells that have been pulsed with viral antigens.

15. Method according to claim 14, wherein the at least one other population of eukaryotic cells comprises Dendritic cells that have been pulsed with viral antigens from a coronavirus, in particular with viral antigens of SARS-CoV-2.
